# FASCICULE DE BREVET EUROPEEN

(11) **EP 0 545 833 B1**
(45) Date de publication et mention de la délivrance du brevet: **09.04.1997**
(21) Numéro de dépôt: 92420439.9
(22) Date de dépôt: 26.11.1992
(51) Int. Cl.: A61F 2/38

(54) **Prothèse du genou**
Knieprothese
Knee prosthesis

(30) Priorité: 29.11.1991 FR 9115044
(43) Date de publication de la demande: 09.06.1993
(73) Titulaire: TORNIER SA, F-38330 Saint Ismier (FR)
(72) Inventeur: Oudard, Jean-Loup, F-38330 Saint Nazaire Les Eymes (FR)
(74) Mandataire: Karmin, Roger

(56) Documents cités:
- EP-A- 0 069 683
- EP-A- 0 376 658
- FR-A- 2 288 509
- US-A- 4 822 366
- US-A- 4 985 037

## Description

La présente invention est relative à une prothèse du genou et plus particulièrement, bien que non exclusivement, à une prothèse de reprise, c'est-à-dire utilisée à un patient et qui doit être remplacée pour une raison ou pour une autre. En cas de reprise, il est souvent nécessaire d'avoir des ancrages plus solides des éléments fémoraux et tibiaux, de telle sorte qu'on utilise généralement pour la fixation de l'élément d'articulation une tige centro-médullaire longue et de diamètre adapté.

On peut également utiliser une telle prothèse dès l'origine.

On a décrit dans le brevet français FR-A-2 288 509 une prothèse du genou comprenant deux éléments associés respectivement au fémur et au tibia, le premier constituant la pièce fémorale qui est réalisée sous la forme d'une plaquette incurvée à deux condyles, la plaquette étant associée à une tige centro-médullaire, tandis que le second élément est constitué par le plateau tibial également associé à une tige centro-médullaire. Les tiges centro-médullaires, solidaires des pièces fémorales ou tibia-les, doivent comporter des caractéristiques différentes suivant les cas chirurgicaux de telle sorte que leur longueur, leur diamètre, leur angulation et leur positionnement par rapport au centre doivent varier plus particulièrement pour la pièce fémorale.

Si l'on veut répondre correctement aux cas chirurgicaux considérés, le chirurgien doit disposer d'une multitude d'implants, de telle sorte que cette solution est extrêmement onéreuse.

On connait également d'après le brevet US 4 822 366 une prothèse de genou comprenant un élément articulaire constitué d'un élément fémoral, pourvue de deux condyles séparés par une fente dans laquelle est disposé un rebord en saillie. Ce rebord est destiné à recevoir en appui sur ses pans inclinés un écrou de même profil lors du serrage de la douille au moyen de la tige d'extension. Cette dernière traverse la douille pour que son extrémité filetée coopère avcec l'écrou.

Le rebord en saillie constitue non seulement une face d'appui mais également une butée angulaire pour le maintien transversal et longitudinal de la douille lors de sa fixation par rapport à l'élément fémoral.

Cette prothèse comporte certains inconvénients en ce que la douille ne peut être réglée transversalement ou longitudinalement par rapport à l'élément fémoral.

Les perfectionnements qui font l'objet de la présente invention visent à remédier à ces inconvénients et à permettre la réalisation d'une prothèse à plusieurs composants qui permette d'obtenir économiquement toutes les dimensions désirées d'implants.

A cet effet, la prothèse suivant l'invention est telle que definie dans la revendication 1.

Le dessin annexé, donné à titre d'exemple, permettra de mieux comprendre l'invention, les caractéristiques qu'elle présente et les avantages qu'elle est susceptible de procurer :
Fig. 1 est une vue en perspective éclatée des différents composants d'une prothèse établie conformément à l'invention.

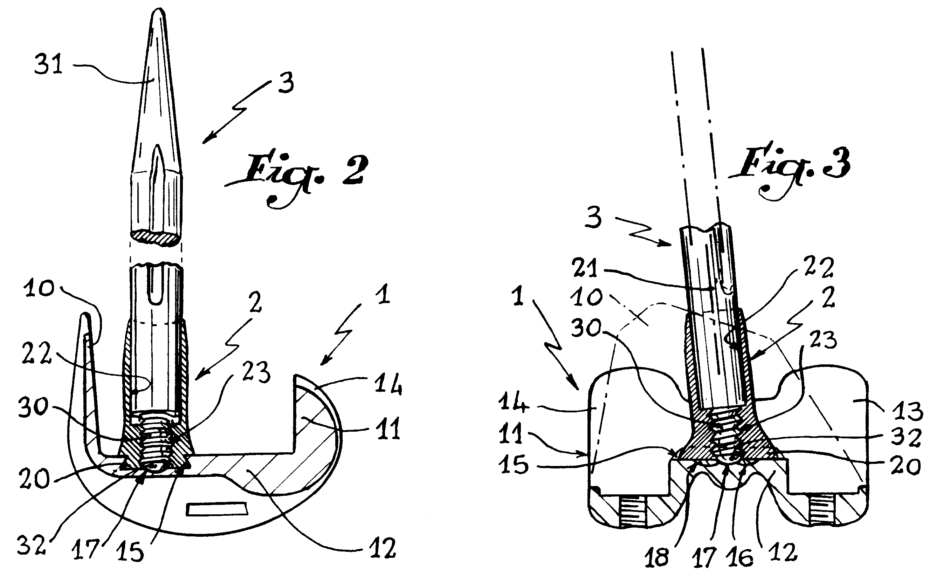

Fig. 2 et 3 sont respectivement des coupes longitudinale et transversale de la prothèse à l'état monté.

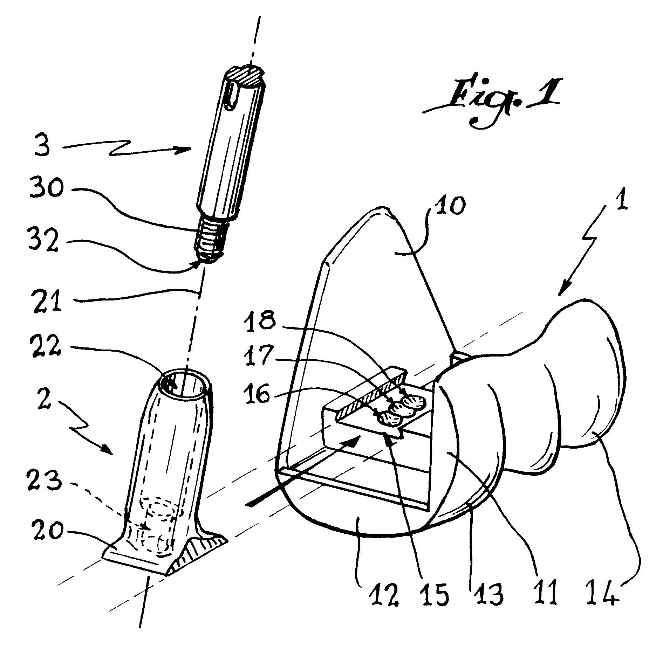

On a illustré en fig. 1 les différents composants d'une prothèse établie conformément à l'invention dont l'élément d'articulation est constitué par une pièce fémorale, restant entendu que l'invention peut aussi bien s'appliquer à un plateau tibial. La pièce fémorale référencée 1 comporte en section transversale la forme générale d'un U, c'est-à-dire qu'elle est formée d'une première aile antérieure 10 et d'une seconde aile postérieure 11, ces deux ailes étant reliées par un voile 12. La face externe des deux ailes et du voile transversal de la pièce fémorale 1 comporte deux condyles latéraux 13, 14.

Conformément à l'invention, la face intérieure du voile 12 est pourvue d'une glissière transversale 15 qui affecte la forme d'une queue d'aronde. Dans le fond de la glissière 15 et suivant son axe géométrique longitudinal, on a ménagé plusieurs empreintes sécantes, trois en l'occurence 16, 17, 18 qui affectent en gros chacune la forme d'une calotte sphérique.

La prothèse suivant l'invention comporte encore une douille 2 pourvue d'une semelle en queue d'aronde 20 dont les dimensions sont telles qu'elle peut s'engager à glissement juste dans la glissière 15 de la pièce fémorale 1. On observe que l'axe géométrique 21 de la douille 2 est oblique par rapport à la base de la semelle 20. La douille comprend un alésage étagé constitué d'un trou lisse 22 et d'un trou taraudé 23 débouchant sur la base de la semelle 20.

Le troisième composant de la prothèse suivant l'invention est une tige centro-médullaire 3 comportant un embout fileté 30 à extrémité bombée 32 propre à se visser dans le trou taraudé 23 de la douille 2. On observe en fig. 2 que la tige centro-médullaire 3 se termine à son extrémité opposée à l'embout 30 par une partie pointue 31.

Pour constituer la prothèse, on engage la semelle 20 de la douille 2 dans la glissière 15, puis on enfile la tige 3 dans la douille 2 jusqu'à ce que son embout 30 vienne au contact du trou taraudé 23. A ce moment, l'embout 30 de la tige 3 est vissé dans ledit trou taraudé 23 jusqu'à ce que l'extrémité bombée 32 de l'embout 30 vienne coopérer avec l'une des empreintes 16, 17, 18. Un serrage efficace assure un assemblage complètement rigide.

La prothèse suivant l'invention permet de répondre de manière simple aux différents besoins chirurgicaux, car chacun de ses composants est choisi en fonction du cas anatomique auquel il faut faire face. C'est ainsi que la tige fémorale 3 présente un diamètre et une longueur adéquats, que la douille est choisie parmi une série suivant la valeur de l'inclinaison de son axe 21 par rapport à sa semelle 20 en fonction de la géométrie de la hanche du patient. Il n'est pas nécessaire de prévoir des douilles droites et gauches puisque chacune est réversible par rapport à la glissière 15 ; enfin il est nécessaire d'avoir une série de pièces fémorales droite et gauche, mais en se limitant à cinq tailles, on résoud pratiquement tous les problèmes. On note enfin la possibilité de régler latéralement la position de la douille, donc de la tige, du fait de la présence de plusieurs empreintes 16, 17, 18 au fond de la glissière 15.

On a ainsi réalisé une prothèse de genou et plus particulièrement, bien que non exclusivement, une prothèse de reprise qui soit susceptible de répondre aux divers desiderata anatomiques avec un nombre de pièces très restreint.

On comprend que la glissière 15, prévue transversale dans la description ci-dessus, pourrait être longitudinale. De plus, il pourrait être ménagé dans le voile 12 deux glissières perpendiculaires. Il va de soi en outre que les perfectionnements suivant la présente invention s'appliquent aussi bien à une pièce fémorale qu'à un plateau tibial.

## Revendications

1. Prothèse du genou, notamment de reprise, comprenant un élément articulaire constitué soit d'une pièce fémorale solidaire d'une tige centro-médullaire associé à une douille, soit d'un plateau tibial solidaire d'une tige centro-médullaire, l'élément articulaire (1) comprenant une glissière (15) dans laquelle est montée la semelle (20) de la douille (2) dont l'axe géométrique (21) est oblique par rapport à la base de la semelle (20), tandis que la tige centro-médullaire (3) comporte des moyens d'assemblage (30,32) à la douille (2) qui coopèrent avec la glissière (15) pour assurer le verrouillage rigide de l'ensemble.

2. Prothèse suivant la revendication 1, caractérisée en ce que la glissière (15) est une rainure en queue d'aronde dont le fond comporte plusieurs empreintes (16, 17, 18) disposées suivant l'axe géométrique de ladite glissière.

3. Prothèse suivant la revendication 1, caractérisée en ce que la glissière (15) de l'élément articulaire (1) est prévue transversale.

4. Prothèse suivant la revendication 1, caractérisée en ce que la glissière (15) de l'élément articulaire (1) est prévue longitudinale.

5. Prothèse suivant la revendication 2, caractérisée en ce que l'alèsage (22) de la douille comporte une partie taraudée (23) dans laquelle se visse un embout fileté (30) de la tige centro-médullaire (3) dont l'extrémité (32) vient en appui dans l'une des empreintes (16, 17, 18) du fond de la glissière.

6. Prothèse suivant la revendication 1, caractérisée en ce qu'elle comprend différents éléments articulaires (1), une série de tiges centro-médullaires (3) de différents diamètres et de longueurs différentes et une pluralité de douilles (2) à inclinaisons variées.

## Claims

1. Knee prosthesis, particularly for revision, comprising an articulation element consisting either of a femoral component integral with an intramedullary rod joined to a bushing, or of a tibial plateau integral with an intramedullary rod, the articulation element (1) comprising a slide (15) in which the sole plate (20) of the bushing (2) is mounted, the geometric axis (21) of which bushing (2) is oblique in relation to the base of the sole plate (20), while the intramedullary rod (3) includes means (30, 32) for joining it to the bushing (2), which cooperate with the slide (15) to ensure the rigid locking of the assembly.

2. Prosthesis according to Claim 1, characterized in that the slide (15) is a dovetailed groove, the bottom of which includes several impressions (16, 17, 18) arranged along the geometric axis of the said slide.

3. Prosthesis according to Claim 1, characterized in that the slide (15) of the articulation element (1) is disposed transversely.

4. Prosthesis according to Claim 1, characterized in that the slide (15) of the articulation element (1) is disposed longitudinally.

5. Prosthesis according to Claim 2, characterized in that the bore (22) of the bushing includes an internally threaded part (23) into which a threaded end-piece (30) of the intramedullary rod (3) is screwed, the end (32) of which bears in one of the impressions (16, 17, 18) in the bottom of the slide.

6. Prosthesis according to Claim 1, characterized in that it comprises various articulation elements (1), a series of intramedullary rods (3) of different diameters and different lengths, and a plurality of bushings (2) of varying inclinations.

## Patentansprüche

1. Knieprothese, insbesondere als Ersatzprothese, die ein Gelenkelement aufweist, das entweder als femoraler Bestandteil, der mit einem mit einer Hülse vereinigten zentro-medullären Zapfen fest verbunden ist, oder als mit einem zentro-medullären Zapfen fest verbundene tibiale Pfanne ausgebildet ist,
**gekennzeichnet durch**
ein Gelenkelement (1) mit einer Gleitschiene (15), in der ein Fuß (20) der Hülse (2) gehaltert ist, deren geometrische Achse zur Fußfläche (20) geneigt ist, wobei der zentro-medulläre Zapfen (3) Verbindungsmittel (30, 32) für die Hülse (2) aufweist, die mit der Gleitschiene (15) zusammenwirken, um eine starre Verriegelung der Anordnung sicherzustellen.

2. Prothese nach Anspruch 1,
**gekennzeichnet durch**
eine Gleitschiene (15) mit einer Schwalbenschwanznut, in deren Führungsfläche mehrere Vertiefungen (16, 17, 18) in Längsrichtung angeordnet sind.

3. Prothese nach Anspruch 1,
**gekennzeichnet durch**
eine transversal ausgerichtete Gleitschiene (15) des Gelenkelements (1).

4. Prothese nach Anspruch 1,
**gekennzeichnet durch**
eine longitudinal ausgerichtete Gleitschiene (15) des Gelenkelements (1).

5. Prothese nach Anspruch 2,
**gekennzeichnet durch**
einen Gewindeabschnitt (23) in der Bohrung (22) der Hülse, in den ein Gewindeabschnitt (30) des zentro-medulären Zapfens (3) schraubbar ist, dessen Ende (32) in eine der Vertiefungen (16, 17, 18) der Führungsfläche der Gleitschiene zur Auflage kommt.

6. Prothese nach Anspruch 1,
**gekennzeichnet durch**
eine Bereitstellung unterschiedlicher Gelenkelemente (1) mittels einer Auswahl aus zentro-medullären Zapfen (3) mit unterschiedlichen Durchmessern und unterschiedlichen Längen, sowie mehrere Hülsen (2) mit unterschiedlichen Neigungen.
